# EUROPÄISCHE PATENTSCHRIFT

(11) **EP 3 514 806 B1**
(45) Veröffentlichungstag und Bekanntmachung des Hinweises auf die Patenterteilung: **02.06.2021**
(21) Anmeldenummer: 18169787.1
(22) Anmeldetag: 27.04.2018
(51) Int. Cl.: H01C 1/084, H01C 1/142, H01C 17/28

(54) **ELEKTRISCHER WIDERSTAND, INSBESONDERE FÜR MEDIZINISCHE IMPLANTATE**
ELECTRICAL RESISTOR, PARTICULARLY FOR MEDICAL IMPLANTS
RÉSISTANCE ÉLECTRIQUE, EN PARTICULIER POUR IMPLANTS MÉDICAUX

(30) Priorität: 23.01.2018 DE 102018101419
(43) Veröffentlichungstag der Anmeldung: 24.07.2019
(73) Patentinhaber: BIOTRONIK SE & Co. KG, 12359 Berlin (DE)
(72) Erfinder: Hauer, Marc, 8610 Uster (CH); Neubauer, Birgit, 72160 Horb (DE); Held, Jochen, 6415 Arth (CH); Henschel, Martin, 13125 Berlin (DE); Pfefferkorn, Thomas, 12557 Berlin (DE); Dettmer, Alexander, 12353 Berlin (DE)
(74) Vertreter: Galander, Marcus

(56) Entgegenhaltungen:
- EP-A1- 0 841 668
- WO-A1-2017/111409
- DE-A1-102013 226 759
- DE-T5-112006 002 516
- US-A1- 2016 343 479

## Beschreibung

Die Erfindung betrifft einen elektrischen Widerstand, insbesondere zur Verwendung als Komponente eines medizinischen Implantats.

Bei einem solchen Implantat kann es sich z. B. um einen implantierbaren Defibrillator handeln. Ein solcher Defibrillator muss in der Lage sein, einen geladenen Kondensator innerhalb einer kurzen Zeitspanne zu entladen, um wieder in einen kontrollierten Ausgangszustand zu gelangen. Der hierfür notwendige elektrische Widerstand muss innerhalb kurzer Zeit hohe Energien aufnehmen. Da der Widerstand insbesondere bei der Endmontage des Defibrillators eingesetzt wird, muss es möglich sein, den Widerstand stoffschlüssig, insbesondere mittels Laserschweißen, zu fügen, um die elektrischen und mechanischen Anforderungen zu erfüllen.

Im Stand der Technik sind diesbezüglich verhältnismäßig aufwendige Prozessschritte vorgesehen. Hierbei wird zunächst der Widerstand aus einem Basismaterial geätzt und lokal mittels Ni und Au galvanisch metallisiert. Mit dieser Metallisierung wird dann ein Anschlussband mittels Widerstandsschweißen verbunden. Anschließend wird die Schweißnaht mit einer Abdeckfolie isoliert.

Weiterhin ist aus der US 9,265,170 B2 ein IC mit einem Schichtaufbau bekannt, wobei ein Verbindungselement für die elektrische und mechanische Anbindung nachträglich angebracht wird.

Hiervon ausgehend liegt der vorliegenden Erfindung die Aufgabe zugrunde, einen elektrischen Widerstand bereitzustellen, der vergleichsweise einfach herstellbar ist und dennoch ein Fügen der Anschlusselemente des Widerstands mittels Schweißen oder Löten erlaubt.

Diese Aufgabe wird durch einen Widerstand mit den Merkmalen des Anspruchs 1 gelöst.

Vorteilhafte Ausgestaltungen des jeweiligen Erfindungsaspekts sind in den entsprechenden Unteransprüchen angegeben und werden nachfolgend beschrieben.

Gemäß Anspruch 1 wird ein elektrischer Widerstand offenbart, mit:
- einem Widerstandsleiter, der auf einer Trägerschicht aufgebracht ist, und
- zwei Anschlusselementen, die elektrisch leitend mit dem Widerstandsleiter verbunden sind.

Erfindungsgemäß ist dabei vorgesehen, dass die beiden Anschlusselemente dazu konfiguriert sind, zum elektrischen Kontaktieren des Widerstands jeweils mit einem elektrischen Kontakt verschweißt oder verlötet zu werden, und dass der Widerstandleiter jeweils einen mit dem jeweiligen Anschlusselement überlappenden Bereich aufweist, der das jeweilige Anschlusselement elektrisch leitend kontaktiert.

Der Widerstandsleiter kann gemäß einer Ausführungsform der Erfindung als eine Beschichtung der Trägerschicht ausgebildet sein. Insbesondere kann der Widerstandsleiter durch Sputterdeposition oder Bedampfen der Trägerschicht mit einem elektrisch leitfähigen Stoff bzw. Material gebildet sein.

Gemäß einer Ausführungsform des erfindungsgemäßen Widerstands ist vorgesehen, dass der Widerstandsleiter auf einer ersten Seite der Trägerschicht angeordnet ist, wobei auf einer der ersten Seite abgewandten zweiten Seite der Trägerschicht eine Metallschicht angeordnet ist, die eine Wärmesenke zur Aufnahme Joulescher Wärme bildet. Die Metallschicht kann direkt auf die zweite Seite der Trägerschicht aufgebracht sein oder kann mit dieser über eine Kleberschicht stoffschlüssig verbunden sein. Die Trägerschicht kann z.B. eine Folie sein. Die Trägerschicht kann z.B. aus einem Kunststoff, einem Polymer oder einem Polyimid bestehen oder kann einen solchen Stoff aufweisen.

Weiterhin ist gemäß einer Ausführungsform des erfindungsgemäßen Widerstands vorgesehen, dass die Anschlusselemente auf der ersten Seite der Trägerschicht angeordnet sind bzw. mit dieser stoffschlüssig verbunden sind.

Weiterhin ist gemäß einer Ausführungsform des erfindungsgemäßen Widerstands vorgesehen, dass die Anschlusselemente Abschnitte der Metallschicht bilden und/oder dass die Anschlusselemente auf der zweiten Seite der Trägerschicht angeordnet sind. Die Anschlusselemente können z.B. über eine Kleberschicht mit der zweiten Seite der Trägerschicht verbunden sein.

Weiterhin ist gemäß einer Ausführungsform des erfindungsgemäßen Widerstands vorgesehen, dass der jeweilige Bereich des Widerstandsleiters eine Durchkontaktierung bildet, wobei sich der jeweilige Bereich jeweils durch eine Durchgangsöffnung in der Trägerschicht hindurch erstreckt und das jeweilige Anschlusselement elektrisch leitend kontaktiert.

Weiterhin ist gemäß einer Ausführungsform des erfindungsgemäßen Widerstands vorgesehen, dass auf den jeweiligen Bereich des Widerstandsleiters zur lokalen Widerstandsabsenkung eine Metalllage aufgebracht ist, so dass der jeweilige Bereich zwischen der jeweiligen Metalllage und dem jeweiligen Anschlusselement angeordnet ist. Die jeweilige Metalllage kann z.B. aus Cu bestehen oder Cu aufweisen. Die jeweilige Metalllage kann durch Abscheiden eines leitfähigen Materials (z.B. Cu) auf dem jeweiligen Bereich gebildet sein.

Weiterhin ist gemäß einer Ausführungsform des erfindungsgemäßen Widerstands vorgesehen, dass die Metallschicht bzw. Wärmesenke einen der folgenden Stoffe aufweist oder aus einem der folgenden Stoffe besteht: Cu; eine Legierung aufweisend Cu und Ni; Ni; Nb; Ta; einen Edelstahl; eine Legierung aufweisend Cu, Ni und Mn; eine Legierung aufweisend 55 Gew.% Cu, 44 Gew.% Ni und 1 Gew.% Mn.

Weiterhin ist gemäß einer Ausführungsform des erfindungsgemäßen Widerstands vorgesehen, dass die beiden Bereiche des Widerstandsleiters über einen mäanderförmigen Abschnitt des Widerstandsleiters (z.B. jeweils integral bzw. einstückig) miteinander verbunden sind.

Weiterhin ist gemäß einer Ausführungsform des erfindungsgemäßen Widerstands vorgesehen, dass der Widerstandsleiter aus einem der folgenden Stoffe besteht oder einen der folgenden Stoffe aufweist: Ti, Au, Cu, Ni, Pd, Nb, Cr.

Weiterhin ist gemäß einer Ausführungsform des erfindungsgemäßen Widerstands vorgesehen, dass der Widerstand eine erste und eine zweite Isolationsschicht aufweist, wobei die erste Isolationsschicht über eine Kleberschicht mit der ersten Seite der Trägerschicht verbunden bzw. verklebt ist und dabei den Widerstandsleiter überdeckt, und wobei die zweite Isolationsschicht über eine Kleberschicht mit der zweiten Seite der Trägerschicht und/oder mit der Wärmesenke verbunden bzw. verklebt ist, wobei die zweite Isolationsschicht die Wärmesenke (bis auf einen Teil der Anschlusselemente) überdeckt. Die beiden Isolationsschichten bilden dabei insbesondere jeweils eine äußerste Deckschicht des Widerstands.

Weiterhin ist gemäß einer Ausführungsform des erfindungsgemäßen Widerstands vorgesehen, dass die Anschlusselemente zwischen den beiden Isolationslagen aus dem Widerstand herausstehen, so dass sie jeweils mit einer weiteren Komponente bzw. einem Kontakt verschweißbar oder verlötbar sind.

Gemäß einem weiteren Aspekt wird ein Verfahren offenbart, aufweisend die Schritte:
- Verbinden einer Trägerschicht mit einer Metallschicht, wobei die Metallschicht eine Wärmesenke des Widerstands sowie zwei Anschlusselemente des Widerstands bildet,
- Ausbilden je einer dem jeweiligen Anschlusselement zugeordneten Durchgangsöffnung, wobei sich die jeweilige Durchgangsöffnung durch die Trägerschicht hindurch hin zum zugeordneten Anschlusselement erstreckt, und
- Aufbringen eines elektrisch leitenden Materials auf eine erste Seite der Trägerschicht zur Ausbildung eines Widerstandsleiters, so dass der Widerstandsleiter sich durch die jeweilige Durchgangsöffnung hin zum jeweiligen Anschlusselement erstreckt und dieses elektrisch leitend kontaktiert, wodurch eine elektrisch leitende Verbindung zwischen dem Widerstandsleiter und den beiden Anschlusselementen hergestellt wird.

Gemäß einem weiteren Aspekt ist vorgesehen, dass das elektrisch leitende Material zur Ausbildung des Widerstandsleiters durch Beschichten der Trägerschicht mit dem Material auf die Trägerschicht aufgebracht wird.

Weiterhin ist gemäß einem weiteren Aspekt vorgesehen, dass das elektrisch leitende Material zur Ausbildung des Widerstandsleiters durch Sputterdeposition des Materials auf die Trägerschicht aufgebracht wird.

Weiterhin ist gemäß einem weiteren Aspekt vorgesehen, dass das elektrisch leitende Material zur Ausbildung des Widerstandsleiters durch Bedampfen der Trägerschicht mit dem Material auf die Trägerschicht aufgebracht wird.

Bei dem elektrisch leitenden Material des Widerstandsleiters kann es sich um einen der folgenden Stoffe handeln bzw. das Material kann einen der folgenden Stoffe aufweisen: Ti, Au, Cu, Ni, Pd, Nb, Cr (siehe auch oben).

Weiterhin ist gemäß einem weiteren Aspekt vorgesehen, dass die Trägerschicht einen der folgenden Stoffe aufweist oder aus einem der folgenden Stoffe besteht: ein Kunststoff, ein Polymer, ein Polyimid. Die Trägerschicht kann insbesondere als Folie ausgebildet sein.

Weiterhin ist vorgesehen, dass die Metallschicht einen der folgenden Stoffe aufweist oder aus einem der folgenden Stoffe besteht (siehe auch oben): Cu, eine Legierung aufweisend Cu und Ni; Ni; Nb; Ta; einen Edelstahl; eine Legierung aufweisend Cu, Ni und Mn; eine Legierung aufweisend 55 Gew.% Cu, 44 Gew.% Ni und 1 Gew.% Mn (diese Legierung wird auch als Konstantan bezeichnet).

Weiterhin ist gemäß einem weiteren Aspekt vorgesehen, dass die Metallschicht als ein Blech ausgebildet ist, das mit der Trägerschicht bzw. Folie verklebt wird.

Weiterhin kann gemäß einem weiteren Aspekt vorgesehen sein, dass zur Widerstandsabsenkung jeweils eine Metalllage auf einen Bereich des Widerstandsleiters aufgebracht wird, der sich in der jeweiligen Durchgangsöffnung zur Metallschicht bzw. zu dem jeweiligen Anschlusselement hin erstreckt und dieses elektrisch kontaktiert. Die jeweilige Metalllage kann durch Abscheiden eines leitfähigen Materials (insbesondere Metall wie z.B. Cu) auf den jeweiligen Bereich gebildet sein.

Weiterhin ist gemäß einem weiteren Aspekt vorgesehen, dass eine erste Isolationsschicht mit der ersten Seite der Trägerschicht verklebt wird, so dass der Widerstandsleiter überdeckt wird, und wobei eine zweite Isolationsschicht mit einer zweiten Seite der Trägerschicht und/oder mit der Metallschicht verklebt wird, so dass die Metallschicht (bis auf je einen Abschnitt der Abschlusselemente) überdeckt wird. Die zweite Seite der Trägerschicht ist insbesondere der ersten Seite der Trägerschicht abgewandt.

Bei der vorstehend beschriebenen Erfindung wird mit Vorteil ein Widerstandsbereich bzw. Widerstandsleiter durch einen Übergangsbereich oder eine Durchkontaktierung direkt mit dem Schweißbereich bzw. den Anschlusselementen verbunden. Hierbei können mehrere Widerstände gleichzeitig/parallel hergestellt werden. Darüber hinaus kann durch den Wegfall der Mischverbindung aus Basismaterial und Anschlussband auf eine kostspielige galvanische Ni/Au-Abscheidung und einen aufwendigen Schweißprozess verzichtet werden. Hierdurch kann wiederum auf eine zusätzliche Isolationslage und einen entsprechenden Isolationstest verzichtet werden.

Weiterhin erlaubt die Erfindung mit Vorteil den Einsatz verschiedener Stoffe bzw. Metalle als Widerstandsleiter (wie z.B. Ti, Au, Cu, Ni, Pd, Pt, Nb, ...). Der Einsatz von alternativen Materialien, welche mit steigender Temperatur einen steigenden elektrischen Widerstand aufweisen, sorgt mit Vorteil für eine adaptive Strombegrenzung innerhalb des Widerstandsleiters während der Kondensatorentladung eines Defibrillators. Die Strombegrenzung wirkt sich wiederum günstig auf die weitere Temperaturerhöhung und somit auf die Stabilität des Widerstandsleiters und der umgebenden Materialien aus.

Im Folgenden sollen Merkmale und Ausführungsformen der Erfindung anhand der Figuren erläutert werden. Es zeigen:
- Fig. 1: eine schematische Schnittdarstellung einer Ausführungsform eines erfindungsgemäßen Widerstands;
- Fig. 2: eine perspektivische Ansicht einer weiteren Ausführungsform eines erfindungsgemäßen Widerstands; und
- Fig. 3: eine schematische Schnittdarstellung des in der Figur 2 gezeigten Widerstands.

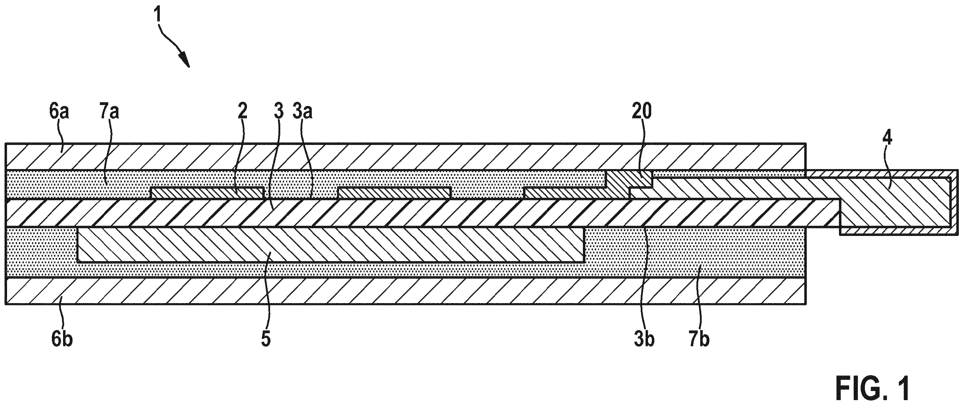

Figur 1 zeigt eine schematische Schnittdarstellung eines erfindungsgemäßen elektrischen Widerstands, insbesondere zur Verwendung in einem implantierbaren Defibrillator.

Der Widerstand 1 weist einen Widerstandsleiter 2 auf, der auf einer Trägerschicht 3 aufgebracht ist, und zwei Anschlusselemente 4, die elektrisch leitend mit dem Widerstandsleiter 2 verbunden sind. Erfindungsgemäß ist vorgesehen, dass die beiden Anschlusselemente 4 dazu konfiguriert sind, zum elektrischen Kontaktieren des Widerstands 1 jeweils mit einem elektrischen Kontakt verschweißt (insbesondere durch Laserschweißen) oder verlötet zu werden, und dass der Widerstandsleiter 2 jeweils einen mit dem jeweiligen Anschlusselement 4 überlappenden Bereich 20 aufweist, der das jeweilige Anschlusselement 4 elektrisch leitend kontaktiert. In der Figur 1 ist aufgrund der Schnittdarstellung nur ein Anschlusselement 4 gezeigt. Das zweite Anschlusselement liegt in einer zur Darstellungsebene parallelen Ebene und wird in gleicher Weise durch einen Bereich des Widerstandsleiters 2 überlappt.

Zur Herstellung des Widerstandsleiters 2 kann Titan (Ti) auf der Trägerschicht 3 z.B. mittels eines Dünnfilmprozesses abgeschieden werden. Der Titanwiderstandsleiter 2 kann dabei mit den Anschlusselementen 4, die jeweils als Kupferstruktur ausgebildet sein können, überlappen und so einen nahtlosen Übergang ermöglichen. Alternative Materialien für den Widerstandsleiter 2 sind z.B. Au, Cu, Ni, Pd, Pt, Nb, Cr oder Kombinationen aus diesen Stoffen.

Das jeweilige Anschlusselement 4 kann in einem Bereich z.B. mittels eines Lasers freigestellt werden, so dass dieser als Schweißkontakt bzw. Anschlussband dienen kann. Das jeweilige Element 4 ermöglicht es jeweils einen Schweißkontakt nahtlos aus einer Leiterplatte herauszuführen.

Auf der zweiten Seite 3b der Trägerschicht 3 ist eine Wärmesenke 5 in Form einer Metallschicht (z.B. Cu) vorgesehen, die dazu dient, Wärme, die während eines elektrischen Pulses des Defibrillators entsteht, aufzunehmen.

Weiterhin können als letzter Herstellungsschritt die Metallstrukturen 2, 20, 4 und 5 auf beiden Seiten 3a, 3b der Trägerschicht 3 zur Isolation mit Isolations- bzw. Deckschichten 6a, 6b versehen werden, die je über eine Kleberschicht 7a, 7b stoffschlüssig mit der Trägerschicht 3 verbunden sind. Die Anschlusselemente 4 ragen dabei zwischen den Deckschichten 6a, 6b hervor.

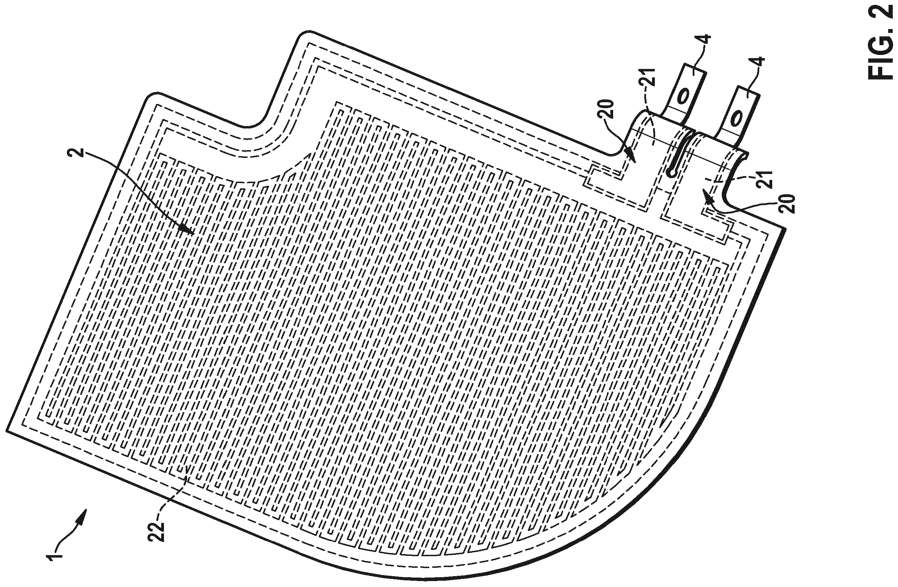

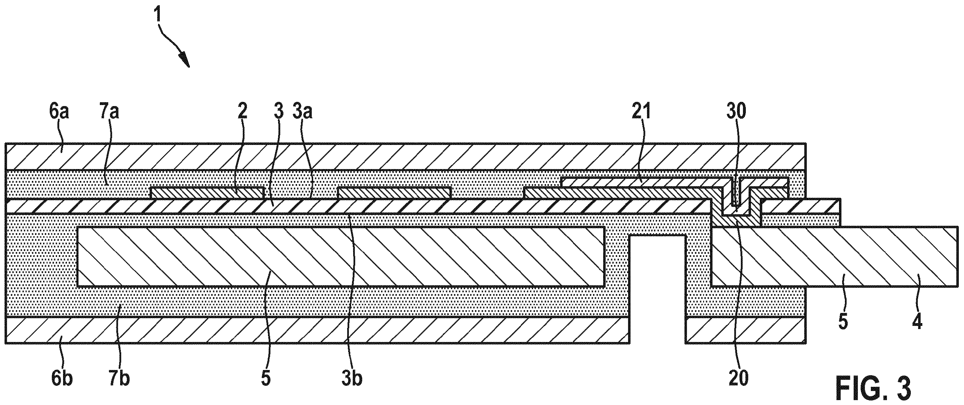

Figur 2 zeigt im Zusammenhang mit der Figur 3 eine weitere Ausführungsform eines erfindungsgemäßen Widerstands 1. In dieser Variante wird auf eine Metallschicht 5 in Form eines Metallblechs 5 aus Konstantan eine Deckfolie 3 aufgeklebt, die eine Trägerschicht 3 bildet. Alternative Materialien für die Metallschicht 5 sind z.B. andere CuNi-Verbindungen, Ni, Nb, Ta, Edelstahl etc. Die Metallschicht 5 dient dabei einerseits als Wärmesenke, andererseits bildet sie schweißbare Anschlusselemente 4. Um eine Verbindung zwischen dem Widerstandsleiter 2 und der Metallschicht 5 herzustellen, wird in der Trägerschicht 3 für jedes Anschlusselement 4 eine Durchgangsöffnung 30 geschaffen (in der Figur 3 ist aufgrund der Schnittdarstellung nur eine Durchgangsöffnung 30 dargestellt, die andere befindet sich in einer zur Darstellungsebene parallelen Ebene), z.B. mittels eines Lasers. Wir nun das Material des Widerstandsleiters 2 (z.B. Titan) auf der ersten Seite 3a der Trägerschicht 3 abgeschieden (z.B. durch Sputterdeposition oder durch Bedampfen), entsteht gleichzeitig jeweils ein Bereich 20 des Widerstandsleiters 2, der in die jeweilige Durchgangsöffnung 30 hineinragt und das jeweils dort freiliegende Anschlusselement 4 bzw. die Metallschicht 5 kontaktiert (Durchgangskontaktierung).

Gemäß einer Ausführungsform der vorliegenden Beschreibung weist die Trägerschicht 3 mehr als eine Durchgangsöffnung 30, wobei zusätzliche Durchgangsöffnungen in Ebenen angeordnet sein können, die parallel zur in Figur 3 dargestellten Ebene sind. Zusätzliche Durchgangsöffnungen können nebeneinander angeordnet sein, wie z.B. neben Durchgangsöffnung 30 in Figur 3. In einer Ausführungsform der Erfindung weist Trägerschicht 3 jeweils 3 Durchgangsöffnungen nebeneinander auf. In einer bevorzugten Ausführungsform weist die Trägerschicht 3 insgesamt 9 Durchgangsöffnungen auf.

Auf dem Titan des jeweiligen Bereiches 20 des Widerstandsleiters 2 kann partiell eine Metalllage 21 (z.B. aus Cu) abgeschieden werden. Dies reduziert lokal den elektrischen Widerstand und ermöglicht es, dass die Leistung nur in dem Bereich abfällt, in dem die Wärme durch die Metallschicht 5 bzw. Wärmesenke aufgenommen werden kann.

Als letzter Schritt können wiederum die Metallstrukturen 2, 20, 21, 4 und 5 auf beiden Seiten 3a, 3b der Trägerschicht 3 zur Isolation mit Isolations- bzw. Deckschichten 6a, 6b versehen werden, die je über eine Kleberschicht 7a, 7b stoffschlüssig mit der Trägerschicht 3 verbunden sind. Die Anschlusselemente 4 ragen dabei wiederum zwischen den Deckschichten 6a, 6b hervor.

In den oben genannten Ausführungsformen kann der Widerstandsleiter eine Schichtdicke im Bereich von 250 nm bis 750 nm, insbesondere 500 nm aufweisen. Weiterhin kann die Trägerschicht 3 jeweils eine Schichtdicke im Bereich von 10 µm bis 40 µm, insbesondere 25 µm, aufweisen. Weiterhin kann die Metallschicht 5 jeweils eine Schichtdicke im Bereich von 35 µm bis 100 µm aufweisen. Weiterhin kann die jeweilige Metalllage 21 eine Schichtdicke im Bereich von 1 µm bis 20 µm aufweisen. Weiterhin können die Anschlusselemente 4 jeweils eine Schichtdicke im Bereich von 35 µm bis 100 µm aufweisen. Weiterhin können die Isolationsschichten 6a, 6b z.B. eine Schichtdicke von 25 µm aufweisen. Schließlich können die Kleberschichten 7a, 7b eine Schichtdicke im Bereich von 25 µm bis 75 µm aufweisen.

Die vorgenannten Werte für die einzelnen Schichtdicken stellen Beispiele der Erfindung dar. Hiervon abweichende Werte können auch möglich sein.

## Patentansprüche

1. Elektrischer Widerstand (1), mit:
- einem Widerstandsleiter (2), der auf einer Trägerschicht (3) aufgebracht ist, und
- zwei Anschlusselementen (4), die elektrisch leitend mit dem Widerstandsleiter (2) verbunden sind,
wobei die beiden Anschlusselemente (4) jeweils mit einem elektrischen Kontakt verschweißbar oder verlötbar sind, und dass der Widerstandleiter (2) jeweils einen mit dem jeweiligen Anschlusselement (4) überlappenden Bereich (20) aufweist, der das jeweilige Anschlusselement (4) elektrisch leitend kontaktiert,
**dadurch gekennzeichnet, dass**
der elektrische Widerstand (1) auf einer ersten Seite (3a) der Trägerschicht und auf einer der ersten Seite (3a) abgewandten zweiten Seite (3b) der Trägerschicht jeweils mit einer Isolationsschicht (6a, 6b) versehen ist, wobei die Anschlusselemente (4) zwischen den Isolationsschichten (6a, 6b) hervorragen oder herausstehen.

2. Elektrischer Widerstand nach Anspruch 1, **dadurch gekennzeichnet, dass** der Widerstandleiter (2) auf der ersten Seite (3a) der Trägerschicht (3) angeordnet ist, wobei auf der der ersten Seite (3a) abgewandten zweiten Seite (3b) der Trägerschicht (3) eine Metallschicht (5) angeordnet ist, die eine Wärmesenke bildet.

3. Elektrischer Widerstand nach Anspruch 1 oder 2, **dadurch gekennzeichnet, dass** die Anschlusselemente (4) auf der ersten Seite (3a) der Trägerschicht (3) angeordnet sind.

4. Elektrischer Widerstand nach Anspruch 2, **dadurch gekennzeichnet, dass** die Anschlusselemente (4) Abschnitte der Metallschicht (5) bilden und/oder dass die Anschlusselemente (4) auf der zweiten Seite (3b) der Trägerschicht (3) angeordnet sind.

5. Elektrischer Widerstand nach Anspruch 1 oder 4, **dadurch gekennzeichnet, dass** der jeweilige Bereich (20) eine Durchkontaktierung bildet, wobei sich der jeweilige Bereich (20) jeweils durch eine Durchgangsöffnung (30) in der Trägerschicht (3) hindurch erstreckt und das jeweilige Anschlusselement (4) elektrisch leitend kontaktiert.

6. Elektrischer Widerstand nach Anspruch 5, **dadurch gekennzeichnet, dass** auf den jeweiligen Bereich (20) zur lokalen Widerstandsabsenkung eine Metalllage (21) aufgebracht ist, so dass der jeweilige Bereich (20) zwischen der jeweiligen Metalllage (21) und dem jeweiligen Anschlusselement (4) angeordnet ist.

7. Elektrischer Widerstand nach einem der Ansprüche 2 bis 6, **dadurch gekennzeichnet, dass** die Metallschicht (5, 4) einen der folgenden Stoffe aufweist oder aus einem der folgenden Stoffe besteht: Cu; eine Legierung aufweisend Cu und Ni; Ni; Nb; Ta; einen Edelstahl; eine Legierung aufweisend Cu, Ni und Mn; eine Legierung aufweisend 55 Gew.% Cu, 44 Gew.% Ni und 1 Gew.% Mn.

8. Elektrischer Widerstand nach einem der vorhergehenden Ansprüche, **dadurch gekennzeichnet, dass** die beiden Bereiche (20) des Widerstandsleiters (2) über einen mäanderförmigen Abschnitt (22) des Widerstandsleiters (2) miteinander verbunden sind.

9. Elektrischer Widerstand nach einem der vorhergehenden Ansprüche, **dadurch gekennzeichnet, dass** der Widerstandsleiter (2) aus einem der folgenden Stoffe besteht oder einen der folgenden Stoffe aufweist: Ti, Au, Cu, Ni, Pd, Nb, Cr.

10. Elektrischer Widerstand nach einem der Ansprüche 2 bis 9, **dadurch gekennzeichnet, dass** der Widerstand (1) eine erste und eine zweite Isolationsschicht (6a, 6b) aufweist, wobei die erste Isolationsschicht (6a) über eine Kleberschicht (7a) mit der ersten Seite (3a) der Trägerschicht (3) verbunden ist und den Widerstandsleiter (2) überdeckt, und wobei die zweite Isolationsschicht (6b) über eine Kleberschicht (7b) mit einer zweiten Seite (3b) der Trägerschicht (3) und/oder mit der Metallschicht (5) verbunden ist, wobei die zweite Isolationsschicht (6b) die Metallschicht (5) zumindest abschnittsweise überdeckt.

## Claims

1. An electrical resistor (1) comprising:
- a resistance conductor (2), which is applied to a carrier layer (3), and
- two connection elements (4), which are electrically conductively connected to the resistance conductor (2),
wherein the two connection elements (4) are configured to each be weldable or solderable to an electrical contact, and in that the resistance conductor (2) for each connection element (4) has a region (20) overlapping the corresponding connection element, which region electrically conductively connects the corresponding connection element (4),
**characterized in that**
the electrical resistor (1) has a first and a second insulation layer (6a, 6b) on a first side (3a) of the carrier layer (3) and on a second side (3b) of the carrier layer (3) facing away from the first side (3a), respectively, wherein the connection elements (4) protrude between the two insulation layers (6a, 6b).

2. The electrical resistor according to claim 1, **characterised in that** the resistance conductor (2) is arranged on a first side (3a) of the carrier layer (3), wherein a metal layer (5) is arranged on a second side (3b) of the carrier layer (3) facing away from the first side (3a) and forms a heat sink.

3. The electrical resistor according to claim 1 or 2, **characterised in that** the connection elements (4) are arranged on the first side (3a) of the carrier layer (3).

4. The electrical resistor according to claim 2, **characterised in that** the connection elements (4) form portions of the metal layer (5) and/or **in that** the connection elements (4) are arranged on the second side (3b) of the carrier layer (3).

5. The electrical resistor according to claim 1 or 4, **characterised in that** the various regions (20) each form a plated through-hole, wherein said regions (20) each extend through a through-opening (30) in the carrier layer (3) and electrically conductively connect the corresponding connection element (4).

6. The electrical resistor according to claim 5, **characterised in that** a metal layer (21) is applied to each of the regions (20) for local resistance reduction, such that each region (20) is arranged between the corresponding metal layer (21) and the corresponding connection element (4).

7. The electrical resistor according to any one of claims 2 to 6, **characterised in that** the metal layer (5, 4) comprises one of the following substances or consists of one of the following substances: Cu; an alloy comprising Cu and Ni; Ni; Nb; Ta; a high-grade steel; an alloy comprising Cu, Ni and Mn; an alloy comprising 55 % by weight Cu, 44 % by weight Ni and 1 % by weight Mn.

8. The electrical resistor according to any one of the preceding claims, **characterised in that** the two regions (20) of the resistance conductor are connected to one another via a meandering portion (22) of the resistance conductor (2).

9. The electrical resistor according to any one of the preceding claims, **characterised in that** the resistance conductor (2) consists of one of the following substances or comprises one of the following substances: Ti, Au, Cu, Ni, Pd, Nb, Cr.

10. The electrical resistor according to any one of claims 2 to 9, **characterised in that** the resistor (1) has a first and a second insulation layer (6a, 6b), wherein the first insulation layer (6a) is connected via an adhesive layer (7a) to the first side (3a) of the carrier layer (3) and covers the resistance conductor (2), and wherein the second insulation layer (6b) is connected via an adhesive layer (7b) to a second side (3b) of the carrier layer (3) and/or to the metal layer (5), wherein the second insulation layer (6b) covers the metal layer (5) at least in some portions.

## Revendications

1. Résistance électrique (1) dotée :
- d'un conducteur de résistance (2) qui est rapporté sur une couche support (3) et
- de deux éléments de connexion (4) qui sont reliés de manière conductrice électriquement avec le conducteur de résistance (2),
dans laquelle les deux éléments de connexion (4) peuvent être soudés ou brasés respectivement avec un contact électrique et où le conducteur de résistance (2) présente respectivement une zone (20) de superposition avec l'élément de connexion (4) respectif qui est en contact de manière conductrice électriquement avec l'élément de connexion (4) respectif,
**caractérisée en ce que**
la résistance électrique (1) est munie respectivement d'une couche isolante (6a, 6b) sur un premier côté (3a) de la couche support et sur un deuxième côté (3b) de la couche support, opposé au premier côté (3a), où les éléments de connexion (4) avancent ou font saillie entre les couches isolantes (6a, 6b).

2. Résistance électrique selon la revendication 1, **caractérisée en ce que** le conducteur de résistance (2) est disposé sur le premier côté (3a) de la couche support (3), où une couche métallique (5) qui forme un puits de chaleur, est disposée sur le deuxième côté (3b) de la couche support (3) opposé au premier côté (3a).

3. Résistance électrique selon la revendication 1 ou la revendication 2, **caractérisée en ce que** les éléments de connexion (4) sont disposés sur le premier côté (3a) de la couche support (3).

4. Résistance électrique selon la revendication 2, **caractérisée en ce que** les éléments de connexion (4) forment des portions de la couche métallique (5) et/ou que les éléments de connexion (4) sont disposés sur le deuxième côté (3b) de la couche support (3).

5. Résistance électrique selon la revendication 1 ou la revendication 4, **caractérisée en ce que** la zone (20) respective forme un contact traversant, où la zone (20) respective s'étend respectivement à travers un orifice de passage (30) dans la couche support (3) et entre en contact de manière conductrice électriquement avec l'élément de connexion (4) respectif.

6. Résistance électrique selon la revendication 5, **caractérisée en ce qu'**une couche métallique (21) est rapportée sur la zone (20) respective pour une diminution de résistance locale de sorte que la zone (20) respective est disposée entre la couche métallique (21) respective et l'élément de connexion (4) respectif.

7. Résistance électrique selon l'une des revendications 2 à 6, **caractérisée en ce que** la couche métallique (5, 4) présente une des substances suivantes ou est constituée d'une des substances suivantes : Cu ; un alliage présentant du Cu et du Ni ; du Ni ; du Nb ; du Ta ; un acier inoxydable, un alliage présentant du Cu, du Ni et du Mn ; un alliage présentant 55 % en poids de Cu, 44 % en poids de Ni et 1 % en poids de Mn.

8. Résistance électrique selon l'une des revendications précédentes, **caractérisée en ce que** les deux zones (20) du conducteur de résistance (2) sont reliées ensemble par le biais d'un segment (22) en forme de méandre du conducteur de résistance (2).

9. Résistance électrique selon l'une des revendications précédentes, **caractérisée en ce que** le conducteur de résistance (2) est constitué d'une des substances suivantes ou présente une des substances suivantes : Ti, Au, Cu, Ni, Pd, Nb, Cr.

10. Résistance électrique selon l'une des revendications 2 à 9, **caractérisée en ce que** la résistance (1) présente une première et une deuxième couche isolante (6a, 6b), où la première couche isolante (6a) est reliée avec le premier côté (3a) de la couche support (3) par le biais d'une couche d'adhésif (7a) et recouvre le conducteur de résistance (2), et où la deuxième couche isolante (6b) est reliée avec le deuxième côté (3b) de la couche support (3) et/ou avec la couche métallique (5) par le biais de la couche d'adhésif (7a), où la deuxième couche isolante (6b) recouvre au moins par endroits la couche métallique (5).
